# FASCICULE DE BREVET EUROPEEN

(11) **EP 1 553 887 B1**
(45) Date de publication et mention de la délivrance du brevet: **18.04.2007**
(21) Numéro de dépôt: 03778424.6
(22) Date de dépôt: 21.10.2003
(51) Int. Cl.: A61B 19/00

(54) **ROBOT CHIRURGICAL D'ORIENTATION ET DE POSITIONNEMENT D'UN INSTRUMENT CHIRURGICAL PORTEUR D'UN OUTIL TERMINAL CHIRURGICAL**
CHIRURGISCHER ROBOTER ZUR ORIENTIERUNG UND POSITIONIERUNG EINES CHIRURGISCHEN INSTRUMENTS MIT EINEM CHIRURGISCHEN WERKZEUG AM ENDE
SURGICAL ROBOT WHICH IS USED TO ORIENT AND POSITION A SURGICAL INSTRUMENT BEARING A SURGICAL END TOOL

(30) Priorité: 22.10.2002 FR 0213169
(43) Date de publication de la demande: 20.07.2005
(73) Titulaire: CENTRE NATIONAL DE LA RECHERCHE SCIENTIFIQUE (CNRS), 75016 Paris (FR); Sinters, 31100 Toulouse (FR)
(72) Inventeur: GIRAUD, Alain, F-31400 Toulouse (FR); ESTEVE, Daniel, F-31520 Ramonville (FR); VAN MEER, Frédérich, F-31500 Toulouse (FR); CARON, Pierre, F-31330 Ondes (FR)
(74) Mandataire: Breese Derambure Majerowicz
(86) Numéro de dépôt international: PCT/FR2003/003118
(87) Numéro de publication internationale: WO 2004/037103

(56) Documents cités:
- EP-A- 0 595 291
- EP-A- 0 700 665
- US-A1- 2002 121 577
- US-B1- 6 406 472

## Description

L'invention concerne un robot chirurgical d'orientation et de positionnement d'un instrument chirurgical porteur d'un outil terminal chirurgical.

La pratique chirurgicale connaît depuis une vingtaine d'années une évolution rapide qui vise à améliorer, d'une part, le confort du patient en réduisant les dimensions des incisions aux strictes nécessités opératoires (outillage, vision et extraction), et d'autre part, le confort du praticien en l'assistant par des procédures de téléopérations robotisées.

Cette pratique à traumatisation minimale, dite « minimale-invasive » a, dans un premier temps, mis en oeuvre des modes opératoires manuels : des petites incisions supportent des trocarts au travers desquels sont introduits les porte-outils, outils de vision, outils chirurgicaux, ... Le chirurgien pratique l'intervention en manipulant directement les outils au travers des trocarts et en contrôlant la réalisation des tâches sur écran de visualisation.

Selon cette pratique, les résultats sont déjà excellents pour le patient qui voit diminuer ses souffrances et réduire sa durée de convalescence. Ce succès a ainsi conduit à ce que de nombreuses opérations sont aujourd'hui pratiquées selon cette technique « minimale-invasive ».

Toutefois, lors de ces procédures manuelles, le travail du chirurgien reste difficile et fatigant. Il doit travailler de longues heures, penché sur le patient en manipulant des outils et porte-outils, regard fixé sur un écran dont l'ergonomie reste encore à améliorer. De plus, le chirurgien subit aussi des fatigues physiques et intellectuelles très importantes qui accroissent les risques d'incidents et même d'accidents opératoires.

Pour pallier ces inconvénients et tirer le meilleur parti de l'approche « minimale-invasive », la solution consiste à assister le chirurgien par des procédures de téléopération robotisée. Dans cette procédure, le chirurgien est placé à distance du patient, équipé d'un poste de vision et de télémanipulation. Il peut aussi télécommander un robot composé de plusieurs bras qui actionne les outils et porte-outils et réalise l'opération.

Chaque bras comporte les constituants suivants :
- un outillage terminal : ciseau, pince, bistouri, ... porté par un instrument chirurgical que l'on peut désolidariser du bras du robot. Les outils sont interchangeables. Le chirurgien a techniquement besoin, en effet, de changer d'outil tout au long de l'opération chirurgicale, ce qui suppose de pouvoir aisément enlever et replacer l'instrument chirurgical et l'outil terminal au travers du trocart,
- un bras robot permettant d'orienter et de positionner l'instrument chirurgical et donc l'outil terminal.

Dans l'acte chirurgical, un point incontournable est de ne pas exercer d'efforts trop importants au niveau de la peau, c'est-à-dire qu'il convient que l'instrument chirurgical s'articule exactement sur un centre de rotation situé au niveau de la peau. Cette exigence fixe donc une spécification incontournable pour le bras robot.

La publication EP 0595 291 A1 divulgue un tel système robotique.

De plus, pour couvrir l'espace de travail souhaité, l'instrument doit, autour du centre de rotation, pouvoir s'incliner de 60° dans toutes les directions et pouvoir pénétrer de 0 à 30 cm sous la peau, tout ceci en conservant une totale maîtrise de l'espace situé au-dessus et à proximité de l'espace de travail. En effet, cette maîtrise de l'espace doit impérativement permettre, notamment :
- le contrôle visuel de l'espace de travail,
- l'utilisation de plusieurs porte-outils coopérant ensemble,
- l'intervention d'outillages manuels,
- l'arrêt d'urgence de l'opération pour des raisons de sécurités médicales,
- la coopération de plusieurs robots travaillant selon des configurations diverses autour du patient.

A l'heure actuelle, quelques premières conceptions sont proposées sur le marché, dérivées de travaux antérieurs réalisés en robotique industrielle : chaque « bras » est constitué d'un robot anthropomorphe classique qui porte les porte-outils et leurs outils. Les premiers essais sont tout à fait encourageants mais ils mettent en évidence des limites et des problèmes non résolus, tels que notamment :
- la disproportion entre les dimensions et la masse des robots proposés (plus de 100 kg) et les dimensions des zones opératoires (quelques décimètres cubes),
- les difficultés de mise en oeuvre liées aux dimensions et aux poids qui impliquent des délais de mise en oeuvre longs et entraînent des coûts prohibitifs,
- les risques encourus par la manipulation de masses importantes à proximité du patient,
- l'encombrement et l'occupation de l'espace situé au-dessus et à proximité de la zone de travail.

La présente invention vise à pallier ces inconvénients et a pour principal objectif de fournir un robot chirurgical d'une part dont les principaux éléments massiques sont déportés de la zone opératoire et d'autre part conçu pour présenter un encombrement et une occupation minimales au-dessus et à proximité de la zone opératoire.

Un autre objectif de l'invention est de fournir des robots chirurgicaux permettant un placement et une coopération facilitées de plusieurs robots autour d'un espace de travail.

A cet effet, l'invention vise un robot chirurgical d'orientation et de positionnement d'un instrument chirurgical porteur d'un outil chirurgical terminal, adapté pour définir un centre de rotation virtuel (c) dudit instrument chirurgical et pour permettre une inclinaison selon toutes les directions de cet instrument autour de ce centre de rotation, et une translation de cet instrument selon des axes passant par ledit centre de rotation.

Selon l'invention, ce robot chirurgical comprend :
- un support de fixation dudit robot (au sol ou sur un mur ou un plafond de bâtiment),
- une potence montée rotative par rapport au support autour d'un axe (X) sécant avec le centre virtuel (c) de rotation de l'instrument,
- des moyens d'entraînement en rotation autour de l'axe (X) de la potence relativement au support,
- un bras porteur de l'instrument chirurgical, constitué d'un système de double parallélogrammes déformables connectés en série, articulé sur la potence de façon à pouvoir pivoter par rapport à cette potence autour d'un axe de rotation adapté, lors de ce pivotement, pour entraîner le pivotement de l'instrument chirurgical autour du centre de rotation virtuel (c) dans une direction complémentaire de celle obtenue par rotation autour de l'axe (X), ledit système de double parallélogramme comportant :
   ■ un premier système de parallélogramme déformable doté de montants longitudinaux montés coulissants, selon un axe de translation parallèle à l'axe de l'instrument, dans des coulisseaux rotatifs par rapport à la potence,
   ■ et un second système de parallélogramme s'étendant en déport longitudinal par rapport au premier système de parallélogramme et doté de montants longitudinaux articulés sur les montants longitudinaux dudit premier système de parallélogramme, et sur les extrémités en déport desquels est articulé un support de l'instrument chirurgical,
- des moyens de déplacement en translation des montants longitudinaux du premier système de parallélogramme relativement aux coulisseaux,
- et des moyens d'entraînement en rotation des coulisseaux relativement à la potence.

Selon le principe de l'invention, le bras du robot portant l'instrument chirurgical consiste en un système de double parallélogrammes, c'est-à-dire en une structure légère constituée de deux systèmes de parallélogrammes connectés en série de façon qu'ils sont portés par une potence déportée et surbaissée pour se situer dans une zone non critique laissant libres la vision et l'accès à la table d'opération.

De plus, tous les mécanismes et motorisations destinés à engendrer les mouvements requis de l'instrument chirurgical, et qui constituent la majeure partie massique du robot, sont également déportés au niveau de cette zone non critique du fait que tous ces mouvements sont commandés et engendrés soit depuis la potence (translation haute/basse et pivotement de l'instrument chirurgical), soit du pied support (rotation de l'instrument chirurgical).

Selon une caractéristique avantageuse de l'invention, les premier et second systèmes de parallélogrammes sont axés dans des plans décalés et parallèles, les montants longitudinaux desdits systèmes de parallélogrammes étant reliés par des traverses.

Cette disposition conduit à déporter la potence non seulement en profondeur mais également latéralement et conduit donc à optimiser la maîtrise de l'occupation de l'espace autour de la zone opératoire. De plus, cette disposition autorise également la réalisation de robots symétriques (déports latéraux respectivement vers la gauche ou la droite) qui favorisent le positionnement de ces robots autour de la table d'opération.

Selon une autre caractéristique avantageuse de l'invention, les moyens d'entraînement en rotation de la potence sont axés dans un plan décalé latéralement par rapport au plan dans lequel est axé le second système de parallélogramme.

Ce décalage de l'axe de motorisation de la potence par rapport à l'axe d'actionnement général du système de double parallélogrammes autorise de placer en parallèle cette motorisation et ce système, et donc de compacter le robot en réduisant son amplitude longitudinale. De plus, ce décalage facilite la coopération de proximité entre deux bras de robots en autorisant de placer leurs outillages terminaux à quelques centimètres l'un de l'autre.

Selon une autre caractéristique avantageuse de l'invention, la distance entre chacune des articulations du support de l'instrument sur un des montants longitudinaux du second système de parallélogramme, et le plan formé par les articulations dudit montant longitudinal sur le premier système de parallélogramme, est non nulle.

Ce décalage entre les articulations du support de l'instrument sur le second système de parallélogramme, par rapport aux articulations de ce second système de parallélogramme sur le premier système de parallélogramme, conduit à permettre d'optimiser la position surbaissée de la potence par rapport à la table d'opération.

Par ailleurs, et de façon avantageuse, les moyens d'entraînement en translation des montants longitudinaux du premier système de parallélogramme comprennent un ensemble pignon/crémaillère associé à chacun desdits montants longitudinaux.

De plus, avantageusement selon l'invention, les moyens d'entraînement en rotation de chaque coulisseau comprennent une poulie libre en rotation autour d'un arbre et solidaire en rotation dudit coulisseau, et des moyens d'entraînement en rotation de ladite poulie.

En outre, chaque ensemble pignon/crémaillère comporte avantageusement un pignon solidaire d'un arbre monté libre en rotation par rapport à la potence et sur lequel est montée libre en rotation la poulie d'entraînement du coulisseau correspondant, les moyens d'entraînement en translation des montants longitudinaux du premier système de parallélogramme comportant, en outre, des moyens d'entraînement en rotation de chaque arbre.

Selon cette disposition avantageuse, un même ensemble d'entraînement permet de commander la translation haut/bas du bras du robot et l'actionnement longitudinal du système de double parallélogramme.

Par ailleurs, de façon avantageuse, le premier système de parallélogramme comprend quatre montants longitudinaux associés deux à deux à un arbre de rotation commun portant les moyens d'entraînement en translation de chacune des dites paires de montants longitudinaux.

Le robot selon l'invention comporte par ailleurs avantageusement un système de contre-poids monté coulissant le long d'un montant parallèle aux montants longitudinaux du premier système de parallélogramme, et relié par un système de renvoi à au moins un arbre rotatif associé à un desdits montants longitudinaux.

Selon l'invention, ce robot chirurgical peut également être avantageusement doté d'actionneurs d'entraînement en rotation de l'instrument chirurgical et d'actionnement de l'outil terminal, montés sur le second système de parallélogramme et reliés à un boîtier de commande et de connexion portant ledit instrument chirurgical par un ensemble de poulies et de câbles.

D'autres caractéristiques, buts et avantages de l'invention ressortiront de la description détaillée qui suit en référence aux dessins annexés qui en représentent, à titre d'exemple non limitatif, un mode de réalisation préférentiel. Sur ces dessins :
- la figure 1 est une vue en perspective d'un robot chirurgical conforme à l'invention,
- la figure 2 est une coupe transversale par un plan vertical A de ce robot chirurgical,
- la figure 3 est une vue de dessus de ce robot chirurgical,
- la figure 4 est une vue de dessus schématique représentant ce robot chirurgical de façon unifilaire.
- et les figures 5 et 6 sont des vues longitudinales représentant de façon unifilaire deux positions différentes de ce robot chirurgical.

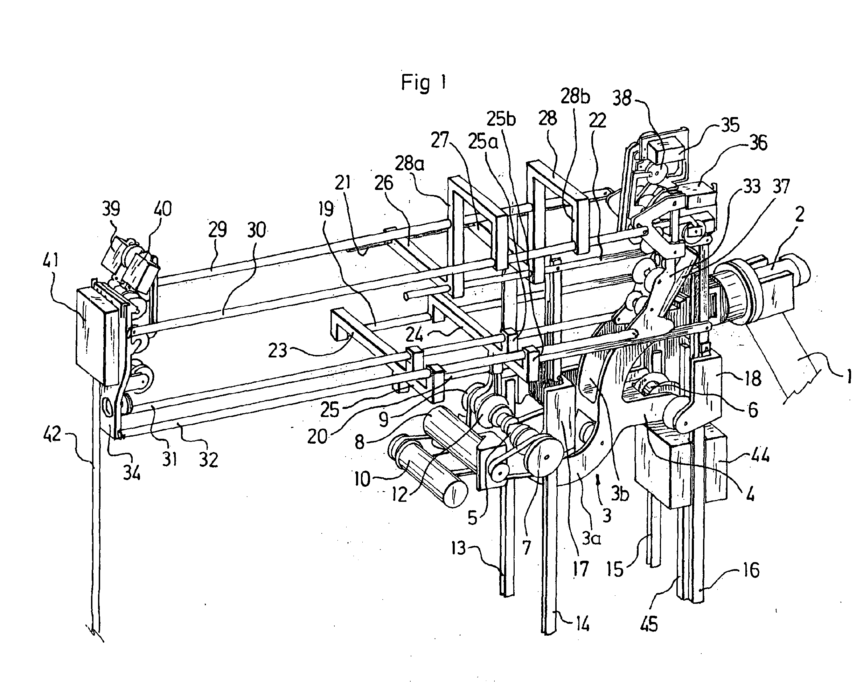

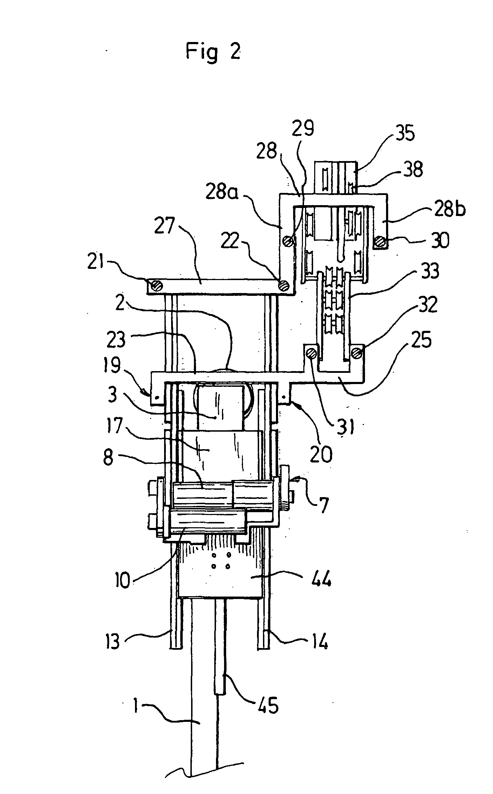

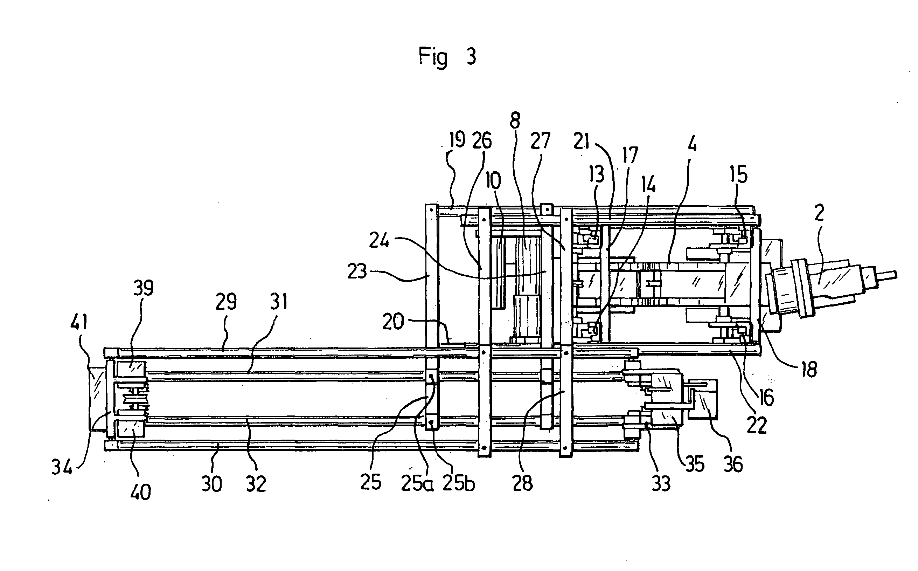

Le robot chirurgical selon l'invention représenté aux figures 1 à 3 est conçu pour permettre d'orienter et de positionner un instrument chirurgical porteur d'un outil chirurgical, lors d'une procédure de téléopération robotisée, et de façon plus spécifique adapté pour définir un centre de rotation virtuel (C) de l'instrument, et pour permettre d'incliner ledit instrument selon toutes les directions autour du centre de rotation (C) et de translater ledit instrument selon des axes passant par ledit centre de rotation.

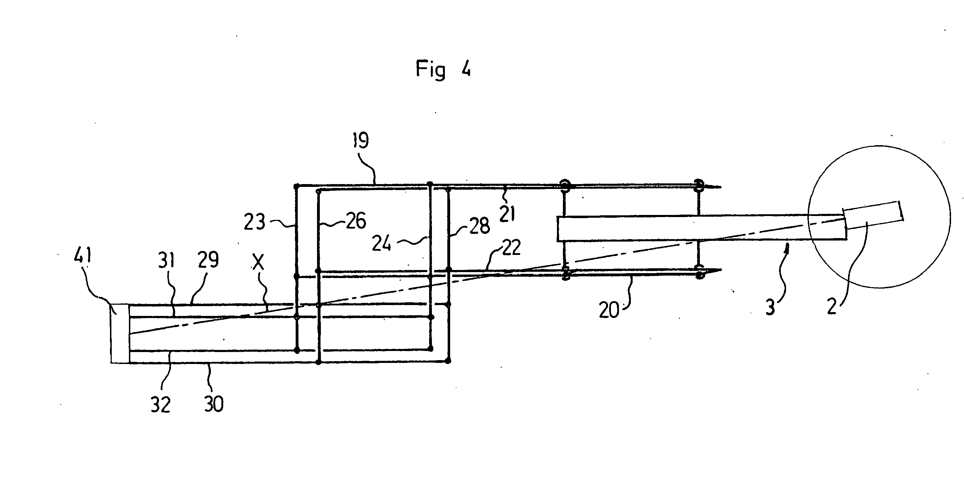

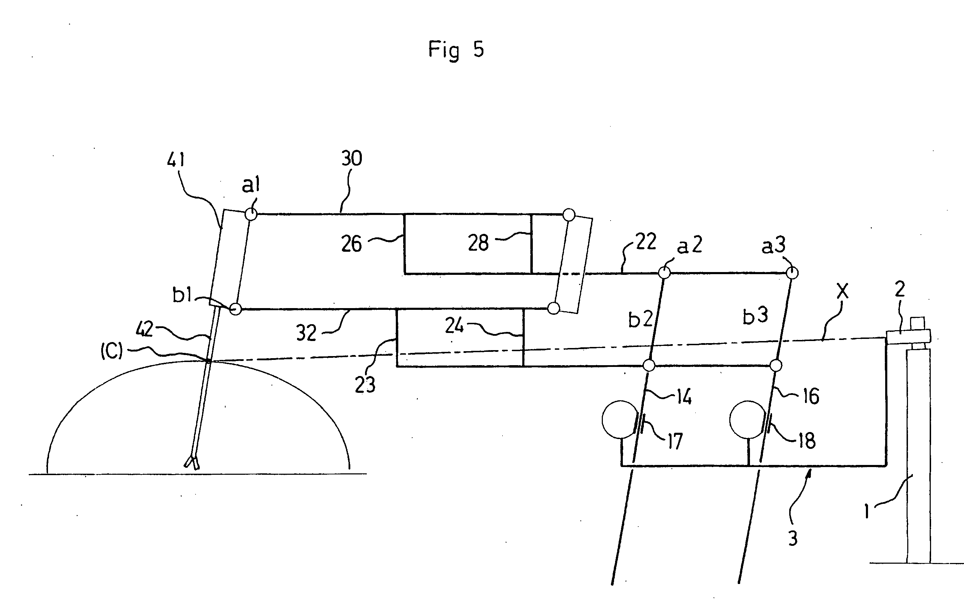

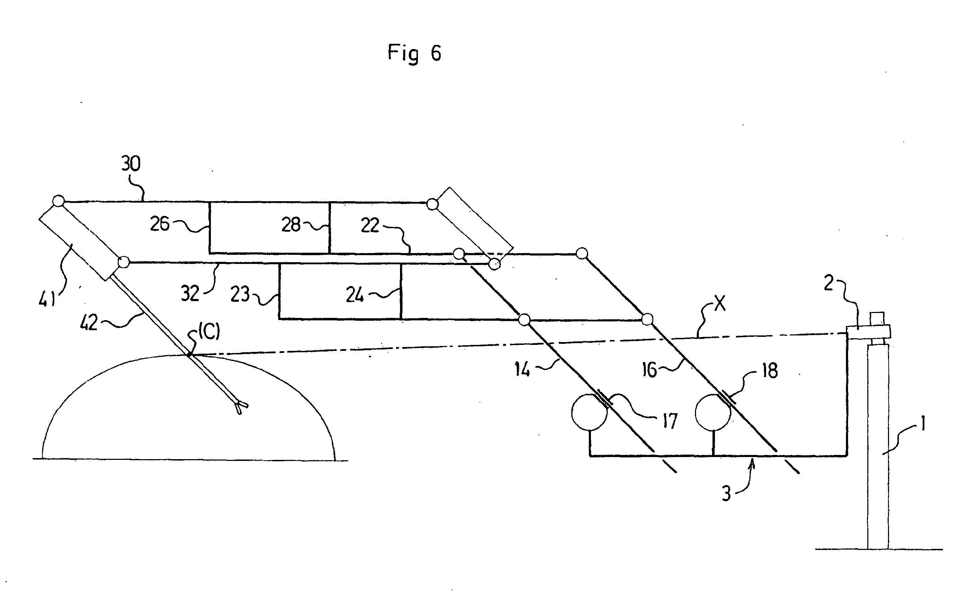

Ce robot chirurgical comprend en premier lieu un pied-support 1 de fixation dudit robot sur le sol, sur lequel est monté un moteur rotatif 2 dont l'axe de rotation est sécant avec le centre de rotation virtuel (C) et, tel que représenté aux figures 4 à 6 incliné de quelques degrés par rapport à la verticale et à l'horizontale. En variante non représentée, le robot peut être supporté par un support solidaire d'un mur ou d'un plafond du bloc chirurgical.

Ce robot chirurgical comprend, en outre, une potence 3 portée par le moteur rotatif 2 mais disposée de façon que ladite potence s'étende verticalement (dans toute la suite de la description, à des fins de clarté de cette description, le robot chirurgical sera décrit tel que représenté à la figure 1, c'est-à-dire avec son bras porteur s'étendant dans un plan vertical, les termes vertical, horizontal,... se référant à cette position).

Cette potence 3 est composée de deux branches parallèles verticales 3a, 3b présentant chacune la forme générale d'un S et reliées par des traverses horizontales. De plus chacune de ces branches 3a, 3b est prolongée vers l'arrière par un bras horizontal 4 disposé sensiblement à mi-longueur de chacune desdites branches.

Cette potence 3 porte deux arbres rotatifs 5, 6 s'étendant horizontalement respectivement entre les extrémités inférieures des branches 3a, 3b et entre les extrémités des bras 4.

Sur le premier de ces arbres 5, porté par les branches 3a, 3b, est montée en premier lieu, au niveau d'une des extrémités dudit arbre, une poulie 7 libre en rotation 7 apte à être entraînée en rotation, par l'intermédiaire d'une courroie, par un moteur 8 s'étendant parallèlement à l'arbre 5 sur un bâti disposé dans le prolongement avant de la potence 3 et solidaire de cette dernière.

Sur chacun des arbres 5 et 6, est également montée une poulie telle que 9 d'entraînement en rotation dudit arbre, disposée au niveau de l'extrémité opposée à celle portant la poulie 7. Ces deux poulies 9 sont entraînées ensemble en rotation, par l'intermédiaire d'une courroie commune, par un moteur 10 s'étendant parallèlement aux arbres 5 et 6 et porté par le bâti précité.

Sur chacun des arbres 5, 6, en position intermédiaire de ces derniers, est enfin monté un pignon 12 solidaire en rotation dudit arbre.

Le robot chirurgical comprend également un bras porteur constitué d'un système de double parallélogrammes connectés en série, articulé sur la potence 3 de façon à pouvoir pivoter autour de l'axe des arbres 5, 6.

Le premier système de parallélogramme de ce bras porteur comprend quatre montants longitudinaux 13-16 sur chacun desquels est fixée longitudinalement une crémaillère.

Ces montants longitudinaux sont portés et montés coulissants deux à deux dans deux berceaux 17, 18 articulés sur la potence 3, de façon que deux desdits montants 13, 14 soient disposés avec leur crémaillère engrainant avec un des pignons 12 du premier arbre 5, et que les deux autres montants 15, 16 soient disposés avec leur crémaillère engrainant avec les pignons 12 du second arbre 6.

Ce premier système de parallélogramme comporte également quatre barres horizontales 19-22 articulées sur les montants longitudinaux 13-16 respectivement au niveau de l'extrémité supérieure de ces derniers, et sensiblement aux trois quarts de leur longueur en partant de leur extrémité inférieure, lesdites barres présentant une longueur adaptée pour s'étendre partiellement dans le prolongement avant des montants 13-16.

Le second parallélogramme de ce robot comporte quant à lui, en premier lieu, quatre barres longitudinales horizontales 29-32 disposées selon les sommets d'un trapèze inversé (petite base inférieure) décalé latéralement par rapport au premier parallélogramme, lesdites barres étant reliées aux barres horizontales 19-22 par quatre traverses :
- deux traverses 23, 24 reliant les deux barres inférieures 31, 32 de ce second parallélogramme aux deux barres horizontales inférieures 19, 20, composées d'un profilé formant au niveau d'une de ses extrémités un U 25 sur chacune des ailes 25a, 25b duquel est portée une barre 31, 32. Ces deux traverses 23, 24 sont en outre disposées, l'une 23 au niveau de l'extrémité des barres 19, 20 et l'autre 24 sensiblement à l'aplomb du premier arbre rotatif 5,
- deux traverses 25, 26 reliant les deux barres supérieures 29, 30 du second parallélogramme aux deux barres horizontales supérieures 21, 22, composées d'un profilé horizontal reliant les deux barres 21, 22, prolongé par une potence 28 en forme de L inversé, dotée de deux ailes verticales 28a, 28b sur chacune desquelles est portée une barre 29, 30. De plus, ces traverses 25, 26 s'étendent sensiblement en retrait vers l'arrière par rapport aux deux premières traverses 23, 24.

Le second parallélogramme comprend, en outre, une armature 33 de liaison de l'extrémité arrière des barres 29-32 sur laquelle lesdites barres sont articulées.

De même, il comprend une plaque 34 de liaison de l'extrémité avant des barres 29-32, sur laquelle lesdites barres sont articulées.

L'armature arrière 33 porte, en outre, des actionneurs tels que 35, 36 d'actionnement de l'outil chirurgical terminal, adaptés pour être reliés à un boîtier 41 de commande et de connexion monté sur la plaque de liaison avant 34, par un système classique de poulies telles que 38 et de câbles s'étendant dans le chemin de câbles que forme le trapèze délimité par les quatre barres 29-32.

Cette armature 33 porte également un moteur 37 d'entraînement en rotation de l'outil terminal autour de l'axe de l'instrument chirurgical.

La plaque de liaison avant 34 porte également deux autres actionneurs 39, 40 d'actionnement de l'outil terminal.

Cette plaque de liaison 34 comporte enfin des moyens classiques de montage amovible et de connexion du boîtier 41 de commande et de connexion sous lequel s'étend l'instrument chirurgical 42.

Le robot chirurgical selon l'invention comporte, enfin, un système de contre-poids, comprenant un poids 44 monté coulissant le long d'un montant 45 disposé entre les deux montants longitudinaux arrières 15, 16 du premier parallélogramme sensiblement en retrait par rapport à ces derniers, et relié par un système de renvoi de type connu en soi à un des arbres rotatifs 5, 6.

Un tel robot chirurgical dont les barres 29-32 du second parallélogramme s'étendent en déport longitudinal par rapport au premier parallélogramme, permet de laisser libres l'accès et la vision à une table d'opération, grâce au regroupement au niveau d'une potence 3 déportée et surbaissée de toutes les commandes d'actionnement du bras du robot.

## Revendications

1. - Robot chirurgical d'orientation et de positionnement d'un instrument chirurgical (42) porteur d'un outil chirurgical terminal, adapté pour définir un centre de rotation virtuel (c) dudit instrument chirurgical et pour permettre une inclinaison selon toutes les directions de cet instrument autour de ce centre de rotation, et une translation de cet instrument (42) selon des axes passant par ledit centre de rotation, ledit robot chirurgical comprenant :
- un support (1) de fixation dudit robot,
- une potence (3) montée rotative par rapport au support (1) autour d'un axe (X) sécant avec le centre virtuel de rotation (c) de l'instrument (42),
- des moyens (2) d'entraînement en rotation autour de l'axe (X) de la potence (3) relativement au support (1),
- un bras porteur de l'instrument chirurgical (42), constitué d'un système de double parallélogrammes déformables connectés en série, articulé sur la potence (3) de façon à pouvoir pivoter par rapport à cette potence autour d'un axe de rotation adapté, lors de ce pivotement, pour entraîner le pivotement de l'instrument chirurgical (42) autour du centre de rotation virtuel (c) dans une direction complémentaire de celle obtenue par rotation autour de l'axe (X), ledit système de double parallélogramme comportant :
■ un premier système de parallélogramme déformable doté de montants longitudinaux (13-16) montés coulissants, selon un axe de translation parallèle à l'axe de l'instrument (42), dans des coulisseaux (17, 18) rotatifs par rapport à la potence (3),
■ et un second système de parallélogramme s'étendant en déport longitudinal par rapport au premier système de parallélogramme (13-16) et doté de montants longitudinaux (29-32) articulés sur les montants longitudinaux (13-16) dudit premier système de parallélogramme, et sur les extrémités en déport desquels est articulé un support (34) de l'instrument chirurgical (42),
- des moyens de déplacement en translation (9, 10, 12) des montants longitudinaux (13-16) du premier système de parallélogramme relativement aux coulisseaux (17, 18),
- et des moyens d'entraînement en rotation (7, 8) des coulisseaux (17, 18) relativement à la potence (3).

2. - Robot chirurgical selon la revendication 1, **caractérisé en ce que** les premier (13-16) et second (29-32) systèmes de parallélogrammes sont axés dans des plans décalés et parallèles, les montants longitudinaux desdits systèmes de parallélogrammes étant reliés par des traverses (23, 24, 26, 27).

3. - Robot chirurgical selon l'une des revendications 1 et 2, **caractérisé en ce que** les moyens d'entraînement en rotation (2) de la potence (3) sont axés dans un plan décalé latéralement par rapport au plan dans lequel est axé le second système de parallélogramme (29-32).

4. - Robot chirurgical selon l'une des revendications 1 à 3, **caractérisé en ce que** la distance entre chacune des articulations (a1-b1) du support (34) de l'instrument (42) sur un des montants longitudinaux (29-32) du second système de parallélogramme, et le plan formé par les articulations (a2, a3-b2, b3) dudit montant longitudinal sur le premier système de parallélogramme (13-16), est non nulle.

5. - Robot chirurgical selon l'une des revendications précédentes, **caractérisé en ce que** les moyens d'entraînement en translation des montants longitudinaux (13-16) du premier système de parallélogramme comprennent un ensemble pignon/crémaillère (12) associé à chacun desdits montants longitudinaux.

6. - Robot chirurgical selon l'une des revendications précédentes, **caractérisé en ce que** les moyens d'entraînement en rotation de chaque coulisseau (17, 18) comprennent une poulie (7) libre en rotation autour d'un arbre (5) et solidaire en rotation dudit coulisseau, et des moyens d'entraînement en rotation (8) de ladite poulie.

7. - Robot chirurgical selon les revendications 5 et 6, **caractérisé en ce que** chaque ensemble pignon/crémaillère comporte un pignon (12) solidaire d'un arbre (5, 6) monté libre en rotation par rapport à la potence (3) et sur lequel est montée libre en rotation la poulie (7) d'entraînement du coulisseau (17, 18) correspondant, les moyens d'entraînement en translation des montants longitudinaux (13-16) du premier système de parallélogramme comportant, en outre, des moyens d'entraînement en rotation (9, 10) de chaque arbre (5, 6).

8. - Robot chirurgical selon la revendication 7, **caractérisé en ce qu'**il comporte un système de contre-poids (44) monté coulissant le long d'un montant (45) parallèle aux montants longitudinaux (13-16) du premier système de parallélogramme, et relié par un système de renvoi à au moins un arbre rotatif (5, 6) associé à un desdits montants longitudinaux.

9. - Robot chirurgical selon la revendication 7, **caractérisé en ce que** le premier système de parallélogramme comprend quatre montants longitudinaux (13-16) associés deux à deux à un arbre de rotation (5, 6) commun portant les moyens d'entraînement en translation (9, 10, 12) de chacune des dites paires de montants longitudinaux.

10. - Robot chirurgical selon l'une des revendications précédentes, **caractérisé en ce qu'**il comprend des actionneurs (35, 36, 39, 40) d'entraînement en rotation de l'instrument chirurgical (42) et d'actionnement de l'outil terminal, montés sur le second système de parallélogramme (29-32) et reliés à un boîtier (41) de commande et de connexion portant ledit instrument chirurgical, par un ensemble de poulies et de câbles (38).

## Claims

1. Surgical robot for orienting and positioning a surgical instrument (42) carrying a surgical end tool, said robot being adapted to define a virtual centre of rotation (c) of the said surgical instrument and to permit inclination of the said surgical instrument about the said centre of rotation in all directions and translation of the said instrument (42) along axes passing through the said centre of rotation, the said surgical robot comprising:
- a support (1) for holding the said robot;
- a bracket (3) which is mounted so as to rotate, in relation to the support (1), about an axis (X) secant with the virtual centre of rotation (c) of the instrument (42);
- means (2) for driving the bracket (3) in rotation about the axis (X), relative to the support (1);
- an arm carrying the surgical instrument (42), which arm is made up of a system of deformable double parallelograms connected in series, which is articulated on the bracket (3) in such a way as to be capable of pivoting, in relation to the said bracket, about an axis of rotation which is adapted, when the said pivoting occurs, to drive the pivoting of the surgical instrument (42) about the virtual centre of rotation (c) in a direction complementary to that obtained by rotation about the axis (X), the said double parallelogram system having:
• a first deformable parallelogram system equipped with longitudinal uprights (13-16) mounted so as to slide, along an axis of translation parallel to the axis of the instrument (42), in slides (17, 18) which rotate in relation to the bracket (3);
• and a second parallelogram system extending in a longitudinally offset manner in relation to the first parallelogram system (13-16) and equipped with longitudinal uprights (29-32) which are articulated on the longitudinal uprights (13-16) of the said first parallelogram system, and on the offset ends of which there is articulated a support (34) for the surgical instrument (42);
- means (9, 10, 12) for displacing the longitudinal uprights (13-16) of the first parallelogram system in translation relative to the slides (17, 18);
- and means (7, 8) for driving the slides (17, 18) in rotation relative to the bracket (3).

2. Surgical robot according to Claim 1, **characterised in that** the first (13-16) and second (29-32) systems of parallelograms are centred in staggered and parallel planes, the longitudinal uprights of the said systems of parallelograms being connected by cross-pieces (23, 24, 26, 27).

3. Surgical robot according to one of Claims 1 and 2, **characterised in that** the means (2) for driving the bracket (3) in rotation are centred in a plane which is staggered laterally in relation to the plane in which the second parallelogram system (29-32) is centred.

4. Surgical robot according to one of Claims 1 to 3, **characterised in that** the distance between each of the articulations (a1-b1) of the support (34) for the surgical instrument (42) on one of the longitudinal uprights (29-32) of the second parallelogram system, and the plane formed by the articulations (a2, a3-b2, b3.) of the said longitudinal upright on the first parallelogram system (13-16), is not zero.

5. Surgical robot according to one of the preceding claims, **characterised in that** the means for driving the longitudinal uprights (13-16) of the first parallelogram system in translation comprise a pinion/rack assembly (12) associated with each of the said longitudinal uprights.

6. Surgical robot according to one of the preceding claims, **characterised in that** the means for driving each slide (17, 18) in rotation comprise a pulley (7) which is free in rotation about a shaft (5) and integral in rotation with the said slide, and means (8) for driving the said pulley in rotation.

7. Surgical robot according to Claims 5 and 6, **characterised in that** each pinion/rack assembly has a pinion (12) which is integral with a shaft (5, 6) which is mounted so as to be free in rotation in relation to the bracket (3) and on which the pulley (7) for driving the corresponding slide (17, 18) is mounted so as to be free in rotation, the means for driving the longitudinal uprights (13-16) of the first parallelogram system in translation having, in addition, means (9, 10) for driving each shaft (5, 6) in rotation.

8. Surgical robot according to Claim 7, **characterised in that** it has a counterweight system (44) which is mounted so as to slide along an upright (45) parallel to the longitudinal uprights (13-16) of the first parallelogram system, and is connected, by a return system, to at least one rotating shaft (5, 6) associated with one of the said longitudinal uprights.

9. Surgical robot according to Claim 7, **characterised in that** the first parallelogram system comprises four longitudinal uprights (13-16) which are associated, in pairs, with a common rotating shaft (5, 6) carrying the means (9, 10, 12) for driving each of the said pairs of longitudinal uprights in translation.

10. Surgical robot according to one of the preceding claims, **characterised in that** it comprises actuators (35, 36, 39, 40) for driving the surgical instrument (42) in rotation and for actuating the end tool, which actuators are mounted on the second parallelogram system (29-32) and are connected, by an assembly of pulleys and cables (38), to a control and connection housing (41) carrying the said surgical instrument.

## Patentansprüche

1. Chirurgischer Roboter zur Orientierung und Positionierung eines chirurgischen Instruments (42), das am Ende ein chirurgisches Werkzeug trägt, wobei der Roboter so gestaltet ist, dass er einen virtuellen Rotationsmittelpunkt (c) des genannten chirurgischen Instruments definiert und eine Neigung dieses Instruments in allen Richtungen um diesen Rotationsmittelpunkt und eine Translation dieses Instrumentes (42) entlang der Achsen zulässt, die durch den genannten Rotationsmittelpunkt passieren, wobei der genannte chirurgische Roboter Folgendes umfasst:
- eine Halterung (1) zum Befestigen des genannten Roboters,
- einen Träger (3), der in Bezug auf die Halterung (1) um eine Achse (X) drehbar montiert ist, die den virtuellen Rotationsmittelpunkt (c) des Instruments (42) schneidet,
- Mittel (2), um den Träger (3) relativ zur Halterung (1) um die Achse (X) in Drehung zu versetzen,
- einen Tragarm für das chirurgische Instrument (42), der von einem System von in Reihe geschalteten verformbaren Doppelparallelogrammen gebildet wird, das so am Träger (3) angelenkt ist, dass es in Bezug auf diesen Träger um eine Rotationsachse schwenken kann, so dass das chirurgische Instrument (42) bei dieser Schwenkung um den virtuellen Rotationsmittelpunkt (c) in einer Richtung komplementär zu der in Drehung versetzt wird, die durch die Rotation des genannten Doppelparallelogrammsystems um die Achse (X) erhalten wird, wobei das Doppelparallelogrammsystem Folgendes umfasst:
• ein erstes verformbares Parallelogrammsystem, das mit einem Längsständergerüst (13-16) ausgestattet ist, das entlang einer Translationsachse parallel zur Achse des Instruments (42) gleitend in Führungen (17, 18) montiert ist, die in Bezug auf den Träger (3) rotieren,
• und ein zweites Parallelogrammsystem, das longitudinal in Bezug auf das erste Parallelogrammsystem (13-16) versetzt verläuft und mit einem Längsständergerüst (29-32) ausgestattet ist, das am Längsständergerüst (13-16) des genannten ersten Parallelogrammsystems angelenkt ist und an dessen versetzten Enden eine Halterung (34) des chirurgischen Instrumentes (42) angelenkt ist,
- Mittel zum translationalen Verschieben (9, 10, 12) des Längsständergerüstes (13-16) des ersten Parallelogrammsystems relativ zu den Führungen (17, 18),
- und Mittel (7, 8), um die Führungen (17, 18) relativ zum Träger (3) in Drehung zu versetzen.

2. Chirurgischer Roboter nach Anspruch 1, **dadurch gekennzeichnet, dass** das erste (13-16) und das zweite (29-32) Parallelogrammsystem in versetzten und parallelen Ebenen angeordnet sind, wobei das Längsständergerüst der genannten Parallelogrammsysteme durch Traversen (23, 24, 26, 27) verbunden ist.

3. Chirurgischer Roboter nach einem der Ansprüche 1 und 2, **dadurch gekennzeichnet, dass** die Mittel (2) zum Indrehungversetzen des Trägers (3) in einer Ebene verlaufen, die lateral in Bezug auf die Ebene versetzt ist, in der das zweite Parallelogrammsystem (29-32) angeordnet ist.

4. Chirurgischer Roboter nach einem der Ansprüche 1 bis 3, **dadurch gekennzeichnet, dass** die Distanz zwischen jeder der Anlenkungen (a1-b1) der Halterung (34) des Instruments (42) an einem der Längsständergerüste (29-32) des zweiten Parallelogrammsystems und der Ebene, die durch die Anlenkungen (a2, a3-b2, b3) des genannten Längsständergerüsts am ersten Parallelogrammsystem (13-16) gebildet wird, ungleich null ist.

5. Chirurgischer Roboter nach einem der vorherigen Ansprüche, **dadurch gekennzeichnet, dass** die Mittel zum translationalen Antreiben des Längsständergerüstes (13-16) des ersten Parallelogrammsystems eine Zahnstangen/Ritzel-Baugruppe (12) in Verbindung mit jedem der genannten Längsständergerüste umfassen.

6. Chirurgischer Roboter nach einem der vorherigen Ansprüche, **dadurch gekennzeichnet, dass** die Mittel zum Indrehungversetzen jeder Führung (17, 18) eine Rolle (7) umfassen, die sich frei um eine Welle (5) drehen kann und drehfest mit der genannten Führung ist, und Mittel zum Indrehungversetzen (8) der genannten Rolle.

7. Chirurgischer Roboter nach den Ansprüchen 5 und 6, **dadurch gekennzeichnet, dass** jede Zahnstangen/Ritzel-Baugruppe ein Ritzel (12) einstückig mit einer Welle (5, 6) aufweist, die in Bezug auf den Träger (3) frei rotieren kann und die rotationsfrei an der Antriebsrolle (7) der entsprechenden Führung (17, 18) montiert ist, wobei die Translationsantriebsmittel des Längsständergerüstes (13-16) des ersten Parallelogrammsystems ferner Drehantriebsmittel (9, 10) jeder Welle (5, 6) umfassen.

8. Chirurgischer Roboter nach Anspruch 7, **dadurch gekennzeichnet, dass** er ein Gegengewichtssystem (44) umfasst, das gleitend entlang einer Strebe (45) parallel zum Längsständergerüst (13-16) des ersten Parallelogrammsystems montiert und durch ein Rückholsystem mit wenigstens einer Drehwelle (5, 6) in Verbindung mit einem der genannten Längsständergerüste verbunden ist.

9. Chirurgischer Roboter nach Anspruch 7, **dadurch gekennzeichnet, dass** das erste Parallelogrammsystem vier Längsständergerüste (13-16) umfasst, die jeweils zu zweit mit einer gemeinsamen Drehwelle (5, 6) assoziiert sind, die die Translationsantriebsmittel (9, 10, 12) jedes der genannten Längsständergerüstpaare tragen.

10. Chirurgischer Roboter nach einem der vorherigen Ansprüche, **dadurch gekennzeichnet, dass** er Stellglieder (35, 36, 39, 40) zum Indrehungversetzen des chirurgischen Instrumentes (42) und zum Betätigen des Werkzeugs am Ende umfasst, die am zweiten Parallelogrammsystem (29-32) montiert und durch eine Rollen- und Seilbaugruppe (38) mit einem Steuer- und Anschlussgehäuse (41) verbunden sind, das das genannte chirurgische Instrument trägt.
